(19) Europäisches Patentamt
European Patent Office
Office européen des brevets

(11) **EP 2 066 475 B1**

(12) **EUROPEAN PATENT SPECIFICATION**

(45) Date of publication and mention
of the grant of the patent:
**11.07.2018 Bulletin 2018/28**

(51) Int Cl.:
**B23Q 15/00** *(2006.01)*     **B27L 5/02** *(2006.01)*
**G01B 11/25** *(2006.01)*     **G01B 11/245** *(2006.01)*

(21) Application number: **07836422.1**

(22) Date of filing: **02.08.2007**

(86) International application number:
**PCT/US2007/017217**

(87) International publication number:
**WO 2008/027150 (06.03.2008 Gazette 2008/10)**

(54) **CHARGER SCANNER SYSTEM**

ABTASTSYSTEM FÜR LADER

SYSTÈME DE SCANNEUR CHARGEUR

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **30.08.2006 US 841178 P**

(43) Date of publication of application:
**10.06.2009 Bulletin 2009/24**

(73) Proprietor: **USNR/Kockums Cancar Company
Vancouver, WA 98662 (US)**

(72) Inventor: **ELY, Gary, W.
Canby OR 97013 (US)**

(74) Representative: **Freeman, Jacqueline Carol et al
WP Thompson
138 Fetter Lane
London EC4A 1BT (GB)**

(56) References cited:
**US-A- 4 248 532        US-A- 4 412 297
US-A- 4 884 605        US-A- 5 949 086
US-A- 6 116 306        US-A- 6 116 306
US-A1- 2004 246 473**

**Description**

**Field of the Invention**

The present invention concerns apparatus and methods for analyzing the geometry and other attributes of a log block in three dimensions, more specifically generating a plurality of scan points for collection within a measurement system used to compute the optimum yield axis of the log block.

**Background Art**

The Coe Manufacturing Company, assignee of the present invention, sells equipment relating to the forestry industry, and in particular, machines and equipment that generate veneer sheets from tree logs. Logs are ultimately positioned in a veneer lathe charger and it is desirable to calculate, as close as possible an optimum yield axis of the log veneer when subject to peeling. Current industry practice is to acquire measurements surrounding a log circumference at multiple stationary locations spaced along the length of the log. The measurements are used to obtain only an approximate center of the log in both a lathe charger and veneer lathe prior to the peeling operation.

The logs, blocks, or log blocks as they are often designated can exceed 40 inches (ca. 1m) in diameter and over 106 inches (ca. 2.7 m) in length when received by the veneer lathe. When in the lathe, the block is turned at a high speed and is engaged along its entire length by a lathe knife, which strips the veneer away, ideally in uninterrupted sheets. The sheets are typically 0.015"-0.25" (ca. 0.038 -0.635 cm) thick, and the log is typically turned until the diameter of the block is less than six inches (ca. 15 cm). Breaks in the sheets of the veneer occur most often when the spindles turning the block are not located about an optimum yield axis. Such breaks equate to waste resulting in undesirable cost and expense. Significant research and development has been allocated to improving equipment and methods for extracting veneer from log blocks. The assignee of the present invention has several patents involving such improvements. Such patents include, United States Patent Nos. 6,116,306, issued September 12, 2000 and 4,884,605, issued December 5, 1989, which describe noncontact scanning using triangulation measurement techniques and multiple single point laser scanners for determining diameter cross-sections of the log block.

Quantities of 5 to 32 independent single point scanners are current common practice for a typical eight foot lathe charger. Installation, alignment, cleaning, and maintenance of large quantity independent scanners are costly and inefficient. Although the techniques described in the aforementioned patents for noncontact scanning an approximate center axis is generally effective, the art lacks the ability to calculate an optimum yield axis. Thus, less than a maximum yield is achieved by employing the techniques currently practiced in the industry as described in the above patents. Anything less than an accurate calculation of the log's optimum yield axis creates waste resulting in costs to the overall product.

US 5,949,086 describes a method for measuring characteristic quantities of a log by moving the log at a known speed past a photographing station through a plane of light and using computer apparatus to calculate sequential images of a group of surface points.

US 6,116,306 describes apparatus for positioning log blocks in a lathe charger by determining the geometric center line of the log at rest and scanning it with light scanners while it rotates to calculate optimum yield axis.

**Summary of the Invention**

The present invention is a new and improved method and apparatus for scanning and analyzing a log block profile for determining an optimum yield axis using a series of block profile scanners. The invention is defined by the claims. According to one example embodiment, four block profile scanners are employed. Each block profile scanner is capable of measuring 512 scanned distances.

**Brief Description of the Drawings**

The foregoing and other features of the present invention will become apparent to one skilled in the art to which the present invention relates upon consideration of the following description of the invention with reference to the accompanying drawings, in which:

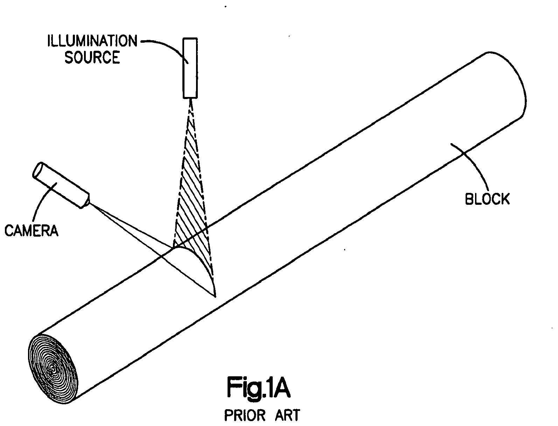

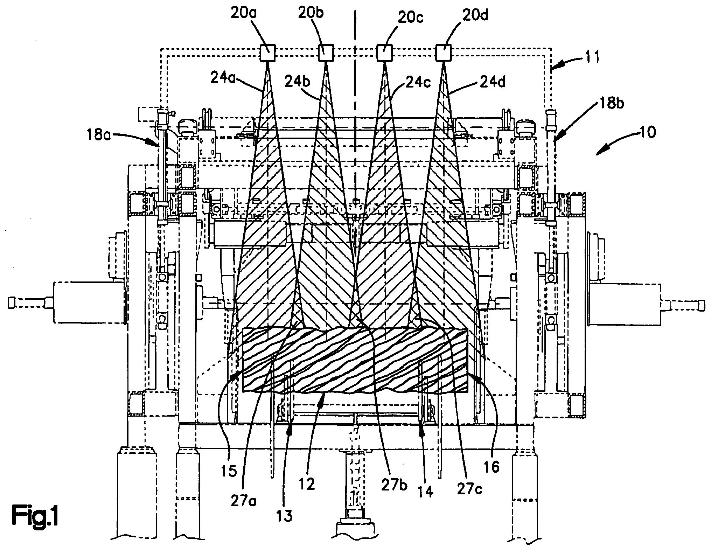

Figure 1A is an isometric view of a scanner used in the prior art;

Figure 1 is a front elevation view of a charger and scanners where the log block is in a first or rest position for a preliminary scanning process;

Figure 2 is a side elevation view of Figure 1 and of a veneer lathe;

Figure 3A is an isometric view showing the operation of a block profile scanner in one example embodiment;

Figure 3B is a diagrammatical view of one example embodiment of a block profile scanner;

Figure 4 is a front elevation view of the charger and scanners where the log block is in a second or yield scan position;

Figure 5 is a partial perspective view of Figure 4;

Figure 6 is a linear representation of a block circumference through 360° as shown in Figure 5;

Figure 7 is a partial front elevation view of a charger positioning a block such that the scanners are used for calculating an optimum yield axis;

Figure 8 is a magnified view of a block exposed to overlapping scanner regions encountering a shadowing condition in the block profile;

Figure 9 is a flow diagram representing a logic process of analyzing a shadowing condition in a log block profile; and

Figure 10 is an illustration representing a filtering process used when scanning a block profile.

## Detailed Description of the Preferred Embodiments

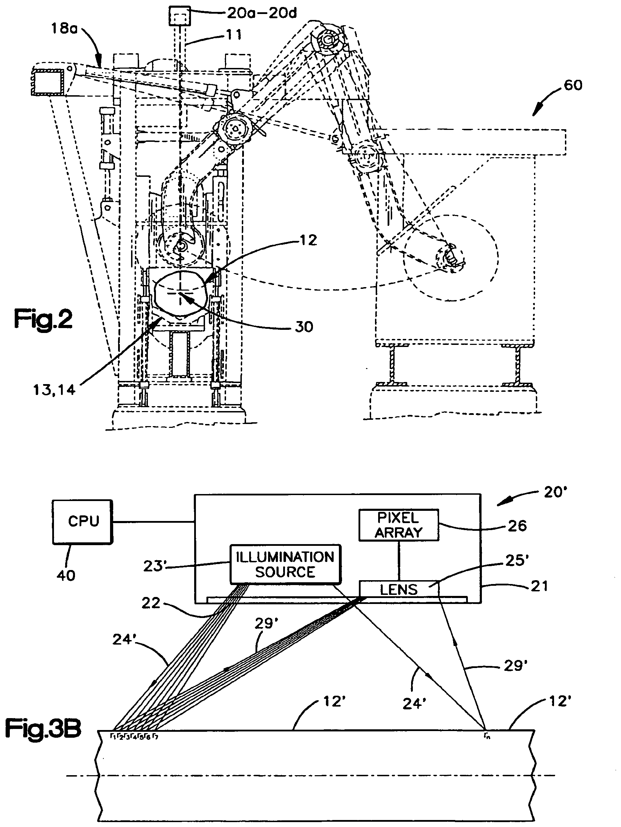

Turning to the drawings, a lathe charger apparatus is shown in phantom generally at 10 in Figures 1 and 2. The lathe charger 10 holds a log block 12 in a rest or first position supported by a first vee 13 and a second vee 14. The block 12 comprises a first end 15 and second end 16, as supported by the vees upon loading into the charger apparatus 10 from a conveyor (not shown). Depicted in Figure 1 are block profile scanners or cameras 20a - 20d suspended above the log block 12 by a fixture 11. Although four scanners 20a - 20d are shown, it should be appreciated by those skilled in the art that any number of scanners can be used without departing from the scope of the claimed invention. A block profile scanner 20 is a triangulation type-measuring scanner capable of capturing a two-dimensional image. The block profile scanner can be for example, a type of scanner that employs a charged couple device (CCD) or complementary metal oxide semiconductor (CMOS) based imaging technology. A suitable block profile scanner will have an illumination source that is projected along the length of the surface of the log block 12, reflecting a surface image profile of the block surface along its length onto a high density two-dimensional pixel array 26.

Figure 1A an isometric view of a scanner system used in the prior art. The scanner system includes an illumination source projecting light along the circumference of a block. The camera or scanner in Figure 1A is limited to imaging the surface of the block at a single circumferential location along the block.

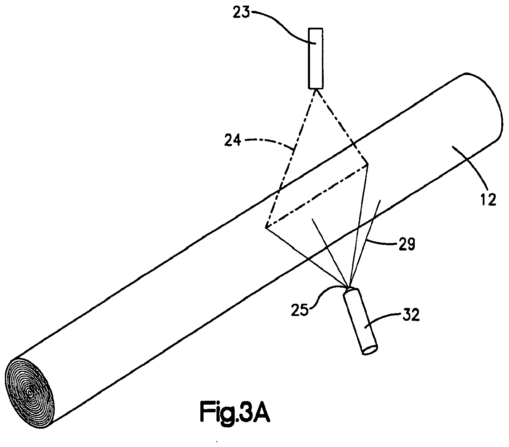

Figure 3A illustrates one example embodiment of a scanner system having an illumination source 23 separated from a camera 32. The illumination source 23 projects a light path 24 upon the block 12. The light path 24 is reflected from the top surface of the block forming a reflected image 29 that is received by a lens 25 on the camera 32. An analysis of the reflected image 29 is performed by a processor (not shown) that can either be internal or external to the camera 32 or scanner 20. A separate example embodiment is shown in Figure 3B. Structures of Figure 3B that are the same or similar to structures of Figure 3A and are labeled with the same reference number with the addition of a prime. Figure 3B features an illumination source 23' and receiving lens 25' contained within a scanner 20'. The reflected image 29' is ultimately projected through the lens 25' onto the high density two-dimensional pixel array 26. In one embodiment, the pixel array has a scan density resolution of 512 x 512 pixels per scanner.

An example of a suitable commercial block profile scanner includes a camera made by IVP Integrated Vision Products Inc. under model number IVP Ranger M20 OEM-1. Further discussion on this type of scanner technology is disclosed in U.S. Patent No. 6,313,876 issued to Eklund. The scanner 20' of Figure 3B further includes a housing 21 with a window 22 from which the illumination source 23' projects a light path 24' upon the surface of the block 12. The illumination source 23' can be one or more light emitting diodes or laser diodes lensed to project a narrow line of light. The light path 24' results in a reflected image 29' of the block surface and is received through a lens 25' located in the scanner 20'. The lens concentrates the reflected image 29' upon the two-dimensional pixel array 26. The data image captured on the pixel array is then analyzed and converted into a value that corresponds to a linear scan distance from the scanner 20 that is associated with a specified scan point or position $r_n$ along the block 12.

Employing a 512 x 512 pixel array, approximately 512 scan distances are measured by each scanner along the profile of the block 12 at rotational increments of the log block at 1.5° or less. Each scan distance is correlated to the simultaneous angle of log block rotation and to the simultaneous distance from scanner face to axis of the log block rotation, thereby providing the three-dimensional data for calculating an optimum yield axis.

## Preliminary Scanning Operation

A preliminary scanning process occurs while the block 12 is in a first position in order to calculate an approximate center 30, as shown in Figure 2. During the preliminary scanning operation, the four block profile scanners 20a - 20d analyze and scan the block 12 from the first end 15 to the second end 16, as best seen in Figure 1.

Each of the block profile scanners generate 512 scan points represented by the values $r_1, r_2, r_3, ... r_n$ in Equation (1) and shown in Figure 3B. The values $r_n$ correspond to a particular pixel in the pixel array 26 that is analyzed and converted into a corresponding scan distance. Each scan distance corresponding to each scan point is collected and stored in a central processing unit (CPU) or computer 40. A variable h of Equation (1) represents a known distance from the scanners less the distance from the calculated scan point. Knowing the values for h and each scan distance corresponding to each scan point $r_n$ along the profile 12 of the block allows for a distance $b_n$ to be calculated, which represents the approximate center 30, as demonstrated by the equations and figure below.

$$h = r_n + b_n \qquad \qquad \textit{Equation (1)}$$

$$r_{approx} = b_n \cos (45°) \qquad \qquad \textit{Equation (2)}$$

$$b_n = h \; / \; 1 + \cos (45°) \qquad \qquad \textit{Equation (3)}$$

## Secondary Scanning Operation for Calculating the Optimum Yield Axis

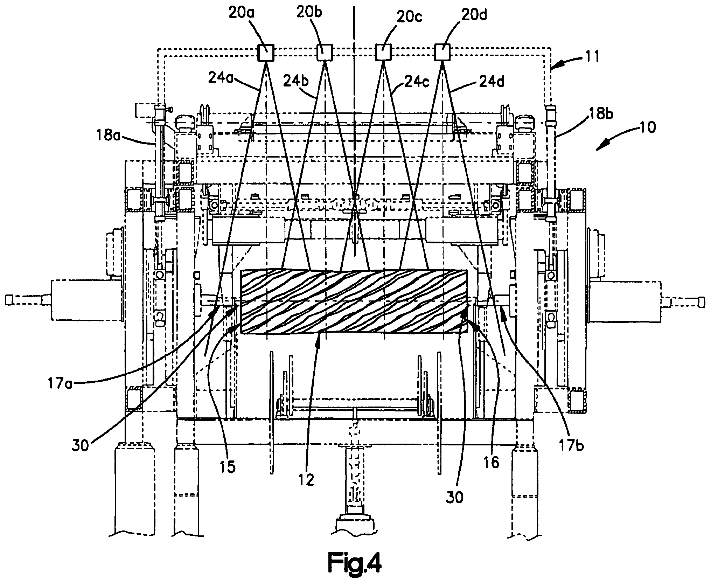

After the completion of the preliminary scan and the calculation of the approximate center 30, a pair of charger spindles shown in Figure 4 as 17a and 17b, are independently moved into engagement with the first and second ends of the log block 12 at the approximate center 30 calculated during the preliminary scanning operation. A pair of hydraulic cylinders 18a and 18b lift block 12 from the first rest position to a second or yield position, as best seen in Figures 4, 5, and 7.

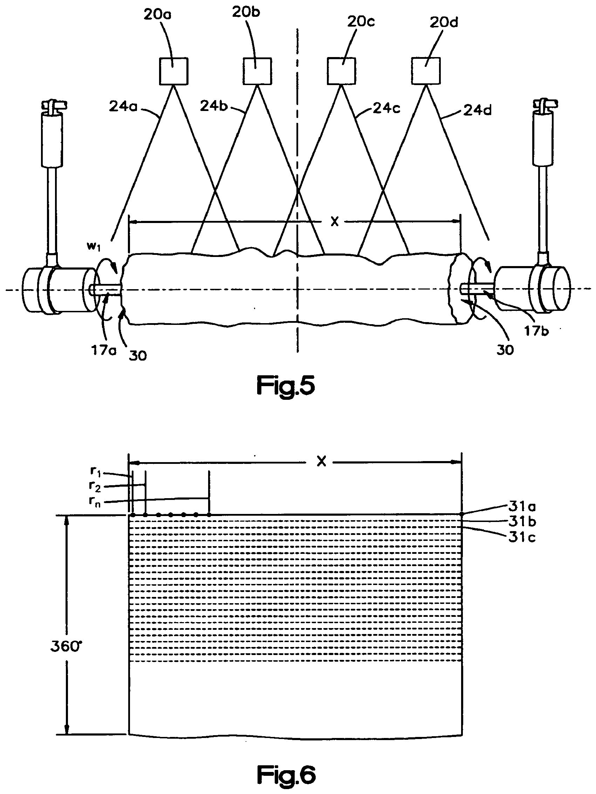

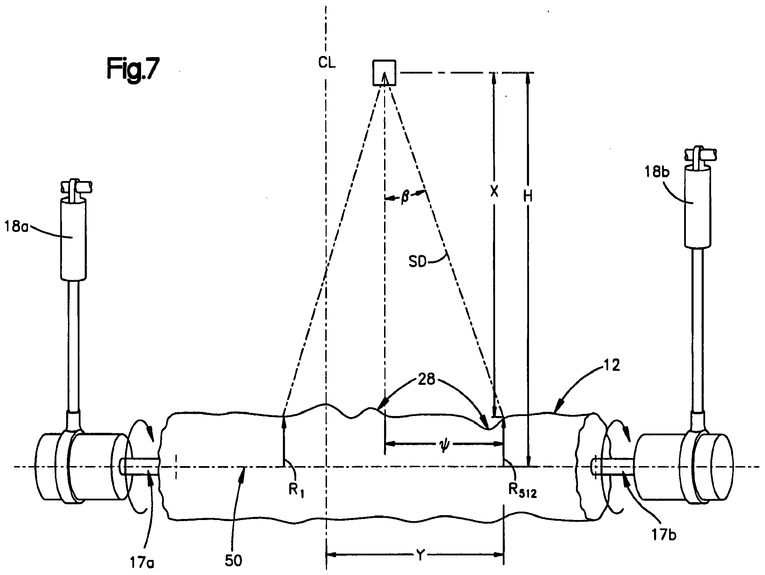

While block lifts from first rest to yield position or while in the yield position, log block 12 assumes an orientation for determining a precise three-dimensional geometric configuration from which the optimum yield axis can be calculated by rotating the log block 12 via charge spindles 17a and 17b. Simultaneously, four light paths represented by 24a - 24d are generated by the illumination source 23 in each respective scanner and are projected along the length profile of the block 12. Each light path contains 512 scan points represented by $r_n$ that provide a reflected image on the pixel array 26, which is equated to a corresponding scan distance to the block 12. The four light paths 24a - 24d combine to form a single scan path 31. As the block rotates, multiple scan paths 31a, 31b... are generated along a longitudinal direction represented by X in Figures 5 and 6 through the 360 degree rotation. Log block 12 is typically accelerated into a spin $\omega_1$ at an approximate rate of 2 revolutions per second and using the described block profile scanners allow for scan paths to occur every 1.5 degrees over the 360 degree rotation of the block 12, thereby generating at least 240 scan paths. The number and frequency of the scan paths could be even greater during the acceleration and deceleration of the block rotation, most typically as the rotational speed decreases. By using four block profile scanners each having 512 x 512 two-dimensional pixel array and generating a scan path along the block approximately every 1.5 degrees results in approximately 419,520 scan points $r_n$ that are measured by the scanner and thus provide a corresponding scan distance SD for each scan point, as shown in Figure 7. Such a significant number of scan points along multiple scan paths provides an elevated scan density, allowing a clear three dimensional analytical depiction of a log block, including detection of any excrescences or discontinuities 28 in the block that often result from knots in the wood. Typically, the larger to the mass of the block 12 the slower the rotational speed, resulting in an even greater number of

scan point $r_n$ than the 419,520 points discussed above.

Knowing the scan distances $SD_n$ based on pixel data from the scan points $r_n$ provide corresponding scan point radii "$R_n$" that collectively form the optimum yield axis of the block 12 represented by line 50 in Figure 7. For example, knowing SD and the height H from the center of the spindle 17 to the corresponding scanner 20, and the angle that a given light beam 24 is projected from scanner center represented by $\beta$ allows the scan point radius $R_n$ to be calculated, which corresponds to a radius of the block at that particular scan point. Although H is a variable distance it is known value, since the distance traveled for each spindle 17 can be tracked by the CPU 40 by, for example encoders (not shown). The scan point radius $R_n$ is the distance from the surface of the block 12 to the optimum yield axis 50 for each scan point $r_n$. This is shown and calculated for scan point radius $R_{512}$ that corresponds to scan point $r_{512}$ in Figure 7 and Equation (4).

$$R_{512} = H - X$$

$$X = \cos(\beta) * SD$$

$$R_{512} = H - \cos(\beta) * SD \qquad\qquad\qquad \textit{Equation (4)}$$

The locus of the scan point radii $R_n$ from the data stream generated by 512 scan points from each scanner along every 1.5 degrees of rotation of the block 12 provides a precise three dimensional shape of log block 12. The three dimensional shape provides approximately 419,520 scan point radii from which the optimum yield axis 50 is calculated. Once the optimum yield axis 50 is computed, such location is used to position block 12 for the highest yield value in the removal of veneer by receiving a pair of pendulum clamps for transferring the log block 12 into a veneer lathe 60 shown generally in phantom in Figure 2. The process executed by the veneer lathe 60 is described in further detail in the '306 patent.

### Scan Path Overlap

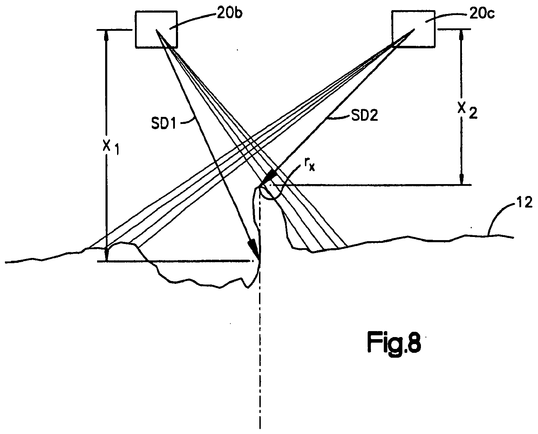

Spacing of the scan points $r_n$ from each scanner along log block length varies with distance from scanner to log surface, typically ranging from 0.050" to 0.100" over the operating range of each scanner. When scanners overlap the result is more dense or closer spaced data and sometimes (with reference to Figures 7 and 8) two X readings may coincidentally apply to the same Y position. An optimizing algorithm 100 will use the smaller indicated radius for calculating optimum yield axis 50. An added advantage of overlap is more accurate surface definition by eliminating shadowing that may result from surface protrusions and recesses.

Each scan distance SD is converted into distance X (perpendicular distance from face of scanner to surface of log as shown in Figures 7 and 8) and distance Y along length of log 12 to a horizontal center location. For each scanner, Y is first expressed as distance from vertical centerline of the scanner, negative to right of center and positive to left. The scanner interface computer 40 combines data from all four scanners and passes all range readings to the optimizing computer in the form of X (perpendicular distance from face of scanners to surface of log) and Y (distance along length of log) with Y=0 at center of material flow, negative Y to right and positive Y to left. For example, (assume X is constant 63"), the last SD at each end of a block 100" long would be reported to the optimizing computer as X = 63", Y = -50" right end and X = 63" and Y = +50" left end.

Shown in Figure 1 are three overlap regions represented by 27a, 27b, and 27c. Each overlap region 27 results from each respective scanner light path 24 extending beyond the adjacent scanner's light path. The amount of surface area of the block 12 subjected to the overlap regions 27 vary depending on the diametrical size and location of the block. The overlapping regions are smaller as the diameter of the block 12 increases or the closer the proximity of the block to the scanner, as depicted when comparing Figure 1 (the block located in a preliminary scan position) with Figure 4 (the block located in a secondary scan position).

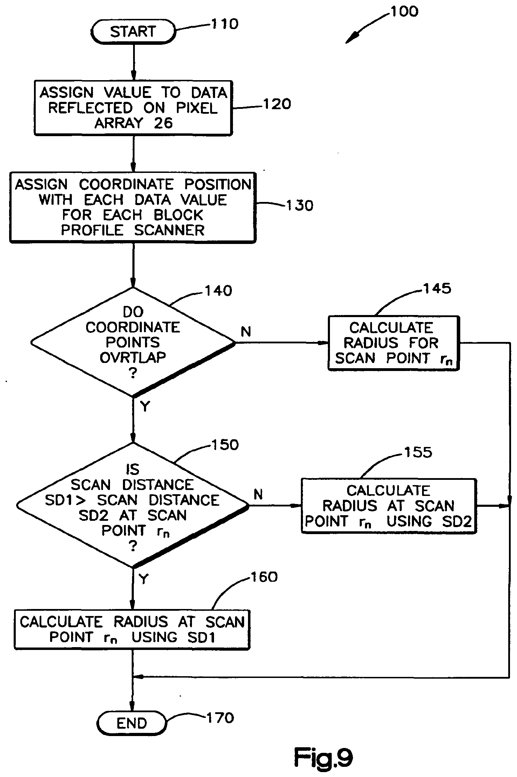

The overlap regions 27 provide an advantage in analyzing areas susceptible to a shadowing condition along the block resulting from projections or protuberances along the block profile as best seen in Figure 8, which is a magnified portion of a log block 12. The overlapping regions, for example in Figure 8 using scanner 20b and 20c provide two different scan distance values for scan point $r_x$ represented by SD1 and SD2, respectively. The optimization algorithm 100 constructed in programs or source code resolves which scan distance to use for overlapping scan points, which is represented generally, by the flow chart in Figure 9.

Referring now to Figure 9, the optimization algorithm source code 100 is initiated at 110. The scan distance reflected onto the pixel array 26 for each scanner is assigned a value at 120 and assigned a coordinate position at 130 relating to the value 120. A decision point 140 evaluates whether the coordinate points are within an overlapping region and assigned the same position. If the points do not overlap the scan point radius $R_n$ of the block 12 is calculated for that particular scan point $r_n$ at 145. If the scan points do overlap and are assigned the same position, a comparison is made at 150. The scan distances are compared for each common scan point at 150. The scan point having the greater scan distance is selected for calculating the block scan point radius $R_n$ at 160. Referring again to Figure 8, SD1 is greater than SD2, therefore the value for SD1 will be used to calculate the scan point radius $R_n$ for scan point $r_x$ in accord with the program 100. As a result, the programming and overlapping regions 27 provide a smaller diameter and more accurate image of the log block 12 when encountering shadowing conditions.

**Steam Penetration Filtering**

The scanning environment frequently includes airborne steam, or any other airborne material, of random intensity between the camera or scanner 20 and the block 12 being scanned. The steam is typically emitted from the block 12 being scanned and/or from peeling veneer from the previously scanned block. Even though laser illumination and reflection from the block surface penetrate the steam, random reflections from the steam can cause multiple extraneous range readings.

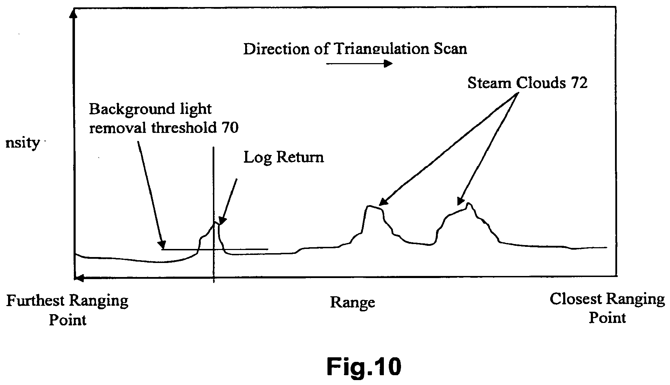

Figure 10 is an illustration representing a filtering process used when scanning a block profile. The cameras 20 are scanned from the farthest range to closest. Only the farthest range reading represents the block surface thus only the farthest reading is used for calculating optimum yield axis. A threshold 70 is provided to remove background ambient light and allow for accurate range measurement. Stated another way, if the scan distance measures an object such as random reflections, airborne material or steam clouds 72, the measured scan distance will be smaller than the threshold 70, and as such, the scan distance measurement is dismissed or filtered from the central processing unit or computer 40 when calculating the optimum yield axis 50.

It is the intent that, although the invention has been described with a degree of particularity, the invention includes all modifications and alterations from the disclosed exemplary embodiment falling within the scope of the appended claims.

**Claims**

1. A measurement system for computing a geometric center of a log block in three dimensions comprising:

   a charger apparatus (10);
   a plurality of block scanner mechanisms (20) having:

   i) an illumination source for projecting light (24) along the length of the surface of the log block onto a plurality of scan points on said surface; and
   ii) an imaging device (32) for receiving an image (29) of the plurality of scan points that is reflected from the surface of the log block; and

   a processing unit (40) configured to determine, based on information from said imaging device (32), a three-dimensional shape of the log block and to calculate an optimum yield axis from said determined three-dimensional shape,
   **characterized in that**
   the block scanner mechanisms (20) are in a spatial alignment such that some of the plurality of scan points of adjacent block scanner mechanisms (20) overlap, and said processing unit (40) further comprises a scan overlap algorithm (100) for selecting only one scan point from each pair of overlapping scan points for use to calculate the optimum yield axis (50).

2. The measurement system of Claim 1, wherein said imaging device (32) is a two-dimensional pixel array.

3. The measurement system of Claim 1, wherein said block scanner (20) is a triangulation type measuring scanner.

4. The measurement system of Claim 1, wherein said scan overlap algorithm uses, from among any two or more of the scan points that overlap, the scan point having the greatest scan distance when determining the three- dimensional shape of the log block to calculate said optimum yield axis (50), and wherein the scan distance of a given scan point is the linear distance from the given scan point to the corresponding block scanner mechanism.

5. The measurement system of Claim 1, wherein said processing unit further comprises a range threshold algorithm such that any scan point having a value less than said threshold is negated when compiling the information representative of the three-dimensional image of the log block to calculate said optimum yield axis (50).

6. The measurement system of Claim 1, wherein said processing unit (40) is a computer.

7. A method for scanning a profile of a log block comprising:

   a) transmitting illumination, from illumination sources located within corresponding first and second block scanners (20), along the length of the surface of said log block;
   b) receiving reflected illumination from along the length of the surface of the log block onto corresponding imaging devices (32) of the first and second block scanners (20) to thereby collect a plurality of scan points;
   c) rotating said log block about a longitudinal axis of the log block while transmitting the illumination from the illumination sources and receiving the reflected illumination;
   d) generating a plurality of scan paths from the scan points collected by the imaging devices;
   e) triangulating the scan points for each of the scan paths; and
   f) computing an optimum yield axis (50) of said block from a numerical model of said block,

   **characterized in that**:

   g) the plurality of scan points includes first and second overlapping scan points collected by the first and second block scanners (20), respectively, and
   h) the method further comprises the step of filtering out only one of the first and second overlapping scan points to produce the numerical model of said block.

8. The method of scanning a profile of a log block in Claim 7, further comprising performing a preliminary scanning process prior to rotating said log block about the longitudinal axis of the log block, wherein performing the preliminary scanning process includes scanning the log block with the first and second block scanners and computing an approximate center of the log.

9. The method of scanning a profile of a log block in Claim 8, wherein the log block remains stationary during the preliminary scanning process.

10. The method of scanning a profile of a log block in Claim 7, further comprising filtering out the scan points having a value less than a range threshold value when triangulating the scan points for each of the scan paths to produce a numerical model of said block.

11. The method of scanning a profile of a log block in Claim 7 wherein filtering out only one of the first and second overlapping scan points includes comparing scan distances of the overlapping scan points and filtering out the overlapping scan point with the smaller of the scan distances, and wherein the scan distance of a given scan point is the distance between the given scan point and the corresponding imaging device.

12. The method of scanning a profile of a log block in Claim 11, further comprising filtering out the scan points having a value less than a range threshold value when triangulating the scan points for each of the scan paths to produce a numerical model of said block.

13. The method of scanning a profile of a log block in Claim 10 wherein filtering out only one of the first and second overlapping scan points includes comparing scan distances of the overlapping scan points and filtering out the overlapping scan point with the smaller of the scan distances, and wherein the scan distance of a given scan point is the distance between the given scan point and the corresponding imaging device.

**Patentansprüche**

1. Messsystem zum Berechnen einer geometrischen Mitte eines Holzblocks in drei Dimensionen, umfassend:

   eine Beschickereinrichtung (10);
   eine Vielzahl von Blockscannermechanismen (20), die Folgendes aufweisen:

   i) eine Beleuchtungsquelle zum Projizieren von Licht (24) entlang der Länge der Oberfläche des Holzblocks auf eine Vielzahl von Scanpunkten auf der Oberfläche; und

ii) eine Abbildungsvorrichtung (32) zum Empfangen eines Bilds (29) von der Vielzahl von Scanpunkten, das von der Oberfläche des Holzblocks reflektiert wird; und

eine Verarbeitungseinheit (40), die konfiguriert ist, auf der Basis von Informationen von der Abbildungsvorrichtung (32) eine dreidimensionale Form des Holzblocks zu bestimmen und um aus der bestimmten dreidimensionalen Form eine Optimalausbeuteachse zu ermitteln,

**dadurch gekennzeichnet, dass** die Blockscannermechanismen (20) räumlich ausgerichtet sind, sodass sich manche der Vielzahl von Scanpunkten benachbarter Blockscannermechanismen (20) überlappen, und dass die Verarbeitungseinheit (40) ferner einen Scanüberlappungsalgorithmus (100) zum Auswählen lediglich eines Scanpunkts aus jedem Paar sich überlappender Scanpunkte zur Verwendung bei der Ermittlung der Optimalausbeuteachse (50) umfasst.

2. Messsystem nach Anspruch 1, wobei die Abbildungsvorrichtung (32) eine zweidimensionale Pixelanordnung ist.

3. Messsystem nach Anspruch 1, wobei der Blockscanner (20) ein Triangulationsmessscanner ist.

4. Messsystem nach Anspruch 1, wobei der Scanüberlappungsalgorithmus aus beliebigen zwei oder mehreren der Scanpunkte, die sich überlappen, den Scanpunkt mit dem größten Scanabstand verwendet, wenn die dreidimensionale Form des Holzblocks bestimmt wird, um die Optimalausbeuteachse (50) zu ermitteln, und wobei der Scanabstand eines bestimmten Scanpunkts der lineare Abstand von dem bestimmten Scanpunkt zu dem entsprechenden Blockscannermechanismus ist.

5. Messsystem nach Anspruch 1, wobei die Verarbeitungseinheit ferner einen Entfernungsschwellenalgorithmus umfasst, sodass jeder beliebige Scanpunkt, der einen geringeren Wert als die Schwelle aufweist, negiert wird, wenn die Informationen zusammengetragen werden, die für das dreidimensionale Bild des Holzblocks repräsentativ sind, um die Optimalausbeuteachse (50) zu ermitteln.

6. Messsystem nach Anspruch 1, wobei die Verarbeitungseinheit (40) ein Computer ist.

7. Verfahren zum Scannen eines Profils eines Holzblocks, umfassend:

a) Übertragen von Beleuchtung von Beleuchtungsquellen, die sich innerhalb eines entsprechenden ersten und zweiten Blockscanners (20) befinden, entlang der Länge der Oberfläche des Holzblocks;

b) Empfangen der reflektierten Beleuchtung von entlang der Länge der Oberfläche des Holzblocks auf entsprechenden Abbildungsvorrichtungen (32) des ersten und zweiten Blockscanners (20), um dadurch eine Vielzahl von Scanpunkten zu sammeln;

c) Drehen des Holzblocks um eine Längsachse des Holzblocks während der Übertragung der Beleuchtung von den Beleuchtungsquellen und Empfangen der reflektierten Beleuchtung;

d) Erzeugen einer Vielzahl von Scanwegen von den Scanpunkten, die von den Abbildungsvorrichtungen gesammelt werden;

e) Triangulieren der Scanpunkte für jeden der Scanwege; und

f) Berechnen einer Optimalausbeuteachse (50) des Blocks aus einem numerischen Modell des Blocks;

**dadurch gekennzeichnet, dass**:

g) die Vielzahl von Scanpunkten einen ersten und zweiten, sich überlappenden Scanpunkt, gesammelt von dem ersten bzw. zweiten Blockscanner (20), beinhaltet und

h) das Verfahren ferner den Schritt des Herausfilterns lediglich eines von dem ersten und zweiten, sich überlappenden Scanpunkt umfasst, um ein numerisches Modell des Blocks herzustellen.

8. Verfahren zum Scannen eines Profils eines Holzblocks in Anspruch 7, ferner umfassend das Durchführen eines vorläufigen Scanvorgangs vor dem Drehen des Holzblocks um die Längsachse des Holzblocks, wobei das Durchführen des vorläufigen Scanvorgangs das Scannen des Holzblocks mit dem ersten und zweiten Blockscanner und das Berechnen einer ungefähren Mitte des Holzes beinhaltet.

9. Verfahren zum Scannen eines Profils eines Holzblocks in Anspruch 8, wobei der Holzblock während des vorläufigen Scanvorgangs stationär bleibt.

**10.** Verfahren zum Scannen eines Profils eines Holzblocks in Anspruch 7, ferner umfassend das Herausfiltern der Scanpunkte, die einen Wert aufweisen, der geringer als ein Entfernungsschwellenwert ist, wenn die Scanpunkte für jeden der Scanwege trianguliert werden, um ein numerisches Modell des Blocks herzustellen.

**11.** Verfahren zum Scannen eines Profils eines Holzblocks in Anspruch 7, wobei das Herausfiltern lediglich eines von dem ersten und zweiten, sich überlappenden Scanpunkt das Vergleichen von Scanabständen der sich überlappenden Scanpunkte und das Herausfiltern des sich überlappenden Scanpunkts mit dem kleineren der Scanabstände beinhaltet, und wobei der Scanabstand eines bestimmten Scanpunkts der Abstand zwischen dem bestimmten Scanpunkt und der entsprechenden Abbildungsvorrichtung ist.

**12.** Verfahren zum Scannen eines Profils eines Holzblocks in Anspruch 11, ferner umfassend das Herausfiltern der Scanpunkte, die einen Wert aufweisen, der geringer als ein Entfernungsschwellenwert ist, wenn die Scanpunkte für jeden der Scanwege trianguliert werden, um ein numerisches Modell des Blocks herzustellen.

**13.** Verfahren zum Scannen eines Profils eines Holzblocks in Anspruch 10, wobei das Herausfiltern lediglich eines von dem ersten und zweiten, sich überlappenden Scanpunkt das Vergleichen von Scanabständen der sich überlappenden Scanpunkte und das Herausfiltern der sich überlappenden Scanpunkte mit dem kleineren der Scanabstände beinhaltet, und wobei der Scanabstand eines bestimmten Scanpunkts der Abstand zwischen dem bestimmten Scanpunkt und der entsprechenden Abbildungsvorrichtung ist.

## Revendications

**1.** Système de mesure servant à calculer un centre géométrique d'un bloc de bille de bois en trois dimensions comportant :

un appareil formant chargeur (10) ;
une pluralité de mécanismes de scanneur de bloc (20) ayant :

i) une source d'éclairage servant à projeter de la lumière (24) sur toute la longueur de la surface du bloc de bille de bois sur une pluralité de points de balayage sur ladite surface ; et
ii) un dispositif d'imagerie (32) servant à recevoir une image (29) de la pluralité de points de balayage qui est réfléchie depuis la surface du bloc de bille de bois ; et

une unité de traitement (40) configurée pour déterminer, en fonction d'informations en provenance dudit dispositif d'imagerie (32) une forme en trois dimensions du bloc de bille de bois et pour calculer un axe de rendement optimum à partir de ladite forme en trois dimensions ayant été déterminée, **caractérisé en ce que** les mécanismes de scanneur de bloc (20) se trouvent dans un alignement spatial de telle sorte que certains de la pluralité de points de balayage de mécanismes de scanneur de bloc adjacents (20) se chevauchent, et ladite unité de traitement (40) comporte par ailleurs un algorithme de chevauchement de balayage (100) servant à sélectionner uniquement un point de balayage en provenance de chaque paire de points de balayage se chevauchant à des fins d'utilisation pour calculer l'axe de rendement optimum (50).

**2.** Système de mesure selon la revendication 1, dans lequel ledit dispositif d'imagerie (32) est un réseau de pixels en deux dimensions.

**3.** Système de mesure selon la revendication 1, dans lequel ledit scanneur de bloc (20) est un scanneur de mesure du type par triangulation.

**4.** Système de mesure selon la revendication 1, dans lequel ledit algorithme de chevauchement de balayage utilise, parmi deux quelconques ou plus des points de balayage qui se chevauchent, le point de balayage ayant la plus grande distance de balayage lors de l'étape consistant à déterminer la forme en trois dimensions du bloc de bille de bois pour calculer ledit axe de rendement optimum (50), et dans lequel la distance de balayage d'un point de balayage donné est la distance linéaire depuis le point de balayage donné jusqu'au mécanisme de scanneur de bloc correspondant.

**5.** Système de mesure selon la revendication 1, dans lequel ladite unité de traitement comporte par ailleurs un algorithme de seuil de plage de telle sorte que tout point de balayage quelconque ayant une valeur inférieure audit seuil est exclu lors de la compilation des informations qui représentent l'image en trois dimensions du bloc de bille de bois pour calculer ledit axe de rendement optimum (50).

**6.** Système de mesure selon la revendication 1, dans lequel ladite unité de traitement (40) est un ordinateur.

**7.** Procédé de balayage d'un profil d'un bloc de bille de bois comportant les étapes consistant à :

a) transmettre un éclairage, en provenance de sources d'éclairage se trouvant dans les premier et deuxième scanneurs de bloc correspondants (20), sur toute la longueur de la surface dudit bloc de bille de bois ;
b) recevoir l'éclairage réfléchi en provenance de toute la longueur de la surface du bloc de bille de bois sur des dispositifs d'imagerie correspondants (32) des premier et deuxième scanneurs de bloc (20) pour de ce fait collecter une pluralité de points de balayage ;
c) faire tourner ledit bloc de bille de bois autour d'un axe longitudinal du bloc de bille de bois tout en transmettant l'éclairage en provenance des sources d'éclairage et recevoir l'éclairage réfléchi ;
d) générer une pluralité de chemins de balayage depuis les points de balayage collectés par les dispositifs d'imagerie ;
e) trianguler les points de balayage pour chacun des chemins de balayage ; et
f) calculer un axe de rendement optimum (50) dudit bloc à partir d'un modèle numérique dudit bloc,

**caractérisé en ce que** :

g) la pluralité de points de balayage comprend des premier et deuxième points de balayage se chevauchant collectés par les premier et deuxième scanneurs de bloc (20), respectivement, et
h) le procédé comporte par ailleurs l'étape consistant à éliminer par filtrage uniquement l'un des premier et deuxième points de balayage se chevauchant pour produire le modèle numérique dudit bloc.

**8.** Procédé de balayage d'un profil d'un bloc de bille de bois selon la revendication 7, comportant par ailleurs l'étape consistant à effectuer un processus de balayage préliminaire avant d'effectuer la rotation dudit bloc de bille de bois autour de l'axe longitudinal du bloc de bille de bois, dans lequel l'étape consistant à effectuer le processus de balayage préliminaire comprend l'étape consistant à balayer le bloc de bille de bois avec les premier et deuxième scanneurs de bloc et l'étape consistant à calculer un centre approximatif de la bille de bois.

**9.** Procédé de balayage d'un profil d'un bloc de bille de bois selon la revendication 8, dans lequel le bloc de bille de bois reste fixe au cours du processus de balayage préliminaire.

**10.** Procédé de balayage d'un profil d'un bloc de bille de bois selon la revendication 7, comportant par ailleurs l'étape consistant à éliminer par filtrage les points de balayage ayant une valeur inférieure à une valeur de seuil de plage lors de la triangulation des points de balayage pour chacun des chemins de balayage pour produire un modèle numérique dudit bloc.

**11.** Procédé de balayage d'un profil d'un bloc de bille de bois selon la revendication 7, dans lequel l'étape consistant à éliminer par filtrage uniquement l'un des premier et deuxième points de balayage se chevauchant comprend l'étape consistant à comparer les distances de balayage des points de balayage se chevauchant et l'étape consistant à éliminer par filtrage le point de balayage se chevauchant ayant la plus petite des distances de balayage, et dans lequel la distance de balayage d'un point de balayage donné est la distance entre le point de balayage donné et le dispositif d'imagerie correspondant.

**12.** Procédé de balayage d'un profil d'un bloc de bille de bois selon la revendication 11, comportant par ailleurs l'étape consistant à éliminer par filtrage les points de balayage ayant une valeur inférieure à une valeur de seuil de plage lors de la triangulation des points de balayage pour chacun des chemins de balayage pour produire un modèle numérique dudit bloc.

**13.** Procédé de balayage d'un profil d'un bloc de bille de bois selon la revendication 10, dans lequel l'étape consistant à éliminer par filtrage uniquement l'un des premier et deuxième points de balayage se chevauchant comprend l'étape consistant à comparer les distances de balayage des points de balayage se chevauchant et l'étape consistant à éliminer par filtrage le point de balayage se chevauchant ayant la plus petite des distances de balayage, et dans lequel la distance de balayage d'un point de balayage donné est la distance entre le point de balayage donné et le dispositif d'imagerie correspondant.

Fig.1A
PRIOR ART

BLOCK

ILLUMINATION
SOURCE

CAMERA

Fig.1

**Fig.2**

**Fig.3B**

Fig.3A

Fig.4

**Fig.5**

**Fig.6**

Fig.7

Fig.8

Fig.9

**Fig.10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6116306 A
- US 4884605 A
- US 5949086 A
- US 6313876 B, Eklund